(19) Europäisches Patentamt
European Patent Office
Office européen des brevets

(11) Veröffentlichungsnummer: **0 057 265**
**B1**

(12) # EUROPÄISCHE PATENTSCHRIFT

(45) Veröffentlichungstag der Patentschrift: **07.03.84**

(51) Int. Cl.³: **C 07 C 59/21**, C 07 C 51/62

(21) Anmeldenummer: **81109026.5**

(22) Anmeldetag: **27.10.81**

(54) **2,2-Dichloracetoacetylchlorid und Verfahren zu dessen Herstellung.**

(30) Priorität: **19.11.80 CH 8549/80**

(43) Veröffentlichungstag der Anmeldung:
**11.08.82 Patentblatt 82/32**

(45) Bekanntmachung des Hinweises auf die Patenterteilung:
**07.03.84 Patentblatt 84/10**

(84) Benannte Vertragsstaaten:
**AT BE CH DE FR GB IT LI LU NL SE**

(56) Entgegenhaltungen:
**DE - C - 1 041 030**
**US - A - 3 007 790**

(73) Patentinhaber: **LONZA AG, Gampel/Wallis (CH)**

(72) Erfinder: **Perlberger, Jean-Claude, Dr., Clos-Louis, Miège (Kanton Wallis) (CH)**

(74) Vertreter: **Weinhold, Peter, Dr. et al, Siegfriedstrasse 8, D-8000 München 40 (DE)**

**EP 0 057 265 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 2,2-Dichloracetoacetylchlorid und Verfahren zu dessen Herstellung

Die Erfindung betrifft 2,2-Dichloracetoacetylchlorid und ein Verfahren für die Herstellung desselben.

2,2-Dichloracetoacetylchlorid ist ein neues Ausgangsmaterial für die spezifische Herstellung der 2,2-Dichloracetessigsäure und ihrer Ester und Amide und von 1,1-Dichloraceton, welche vor allem bei der Formulierung von Insektiziden Verwendung finden.

Es ist bekannt, dass die Chlorierung von Acetylchlorid mit Chlor Chloracetylchlorid als Hauptprodukt und Dichloracetylchlorid als Nebenprodukt liefert. Diese Reaktion verläuft ohne den Einsatz von Katalysatoren nur sehr langsam. Für diese Art von Umsetzungen bekannte Katalysatoren, wie Jod (H.B. Watson und E.E. Roberts - J. Chem. Soc. 2779, 1928), Schwefelsäure (DE-OS 22 63 580) und Chlorsulfonsäure (DE-OS 27 29 911), erlauben zwar eine Unterdrückung der Bildung von Dichloracetylchlorid, fördern aber die Formierung von 2,2-Dichloracetoacetylchlorid nicht, sondern begünstigen vor allem das Entstehen von Chloralkanoylchloriden.

Die Aufgabe der vorliegenden Erfindung war es, ein technisch einfaches Verfahren für die Herstellung der gesuchten Verbindung 2,2-Dichloracetoacetylchlorid zu finden, bei welcher die Entstehung von Nebenprodukten, wie Chloracetylchlorid und Dichloracetylchlorid, möglichst vermieden wird.

Erfindungsgemäss wird die Lösung dieser Aufgabe dadurch erreicht, dass die Chlorierung von Acetylchlorid mit Chlor in Gegenwart einer Lewis-Säure als Katalysator vor sich geht. Als vorteilhaft erwiesen sich anorganische Lewis-Säuren und im besonderen Aluminiumhalogenide, vorzugsweise Aluminiumchlorid $AlCl_3$ und Aluminiumbromid $AlBr_3$. Um ein Optimum an Ausbeute erreichen zu können, erwies es sich als notwendig, die Reaktion bei einer Temperatur von 30 bis 60°C, vorzugsweise bei 40 bis 50°C ablaufen zu lassen, wobei sich das Mol-Verhältnis zwischen Acetylchlorid, dem Katalysator und dem zugeleiteten Chlor von 1 zu 0,005 bis 0,5 zu 0,1 bis 1,5, vorzugsweise von 1 zu 0,03 zu 1,02, als das beste zeigte.

Durch die Chlorierung von Acetylchlorid mit Chlor in Gegenwart einer Lewis-Säure, vorzugsweise von $AlCl_3$ oder $AlBr_3$ als Katalysator wurden nicht zur Hauptsache die zu erwartenden Produkte Chloracetylchlorid und Dichloracetylchlorid erhalten, sondern es konnte, für jeden Fachmann überraschend, die neue, in der Literatur nicht beschriebene Verbindung 2,2-Dichloracetoacetylchlorid in grossem Überschuss und mit guter Ausbeute isoliert werden.

Beispiel 1

In einem mit Rückflusskühler (Trockeneis, Aceton), Thermometer, Magnetrührer und Einleitungsrohr versehenen Rundkolben wurden 39,25 g (0,5 Mol) Acetylchlorid und 2 g (0,015 Mol) Aluminiumchlorid vorgelegt. Das Gemisch wurde auf 45°C erhitzt und während 2,5 Stunden bei dieser Temperatur 36 g (0,51 Mol) Chlor eingeleitet. Die gebildete Salzsäure wurde in Wasser absorbiert und titriert (17,6 g; 0,48 Mol). Die GC-Analyse des Reaktionsgemisches (46,8 g) ergab folgendes:

14,3% Acetylchlorid (AC)
12,0% Chloracetylchlorid (CAC)
1,5% Dichloracetylchlorid (DCAC)
7,0% Trichloracetylchlorid
61,7% 2,2-Dichloracetoacetylchlorid (DCAAC).

Dies entspricht einer Ausbeute von 60,6% bei einer Selektivität von 73%.

Die Aufarbeitung des 2,2-Dichloracetoacetylchlorids resp. dessen Abtrennung aus dem Reaktionsgemisch erfolgte mittels Destillation, zuerst bei Normaldruck, dann bei 0,133 bar (100 Torr) (95 bis 97°C). Die entsprechende Fraktion enthielt das gesuchte 2,2-Dichloracetoacetylchlorid mit einer Reinheit von 98,7%.

Beispiele 2–5

| Beispiel | Katalysator | Acetylchlorid (AC) Umsatz | Selektivität/% | | | Ausbeute % an DCAAC auf eingesetztes AC berech. |
|---|---|---|---|---|---|---|
| | | | CAC | DCAC | DCAAC | |
| 2 | $AlCl_3$ | 83% | 12 | 1,2 | 73 | 60,6 |
| 3 | $AlBr_3$ | 94,5% | 10 | 0,4 | 73 | 69,0 |
| 4 | $SbCl_3$ | 88% | 39 | 3,5 | 31 | 27,3 |
| 5 | Jod | 38% | 99 | 1,0 | 0 | 0 |

Die Beispiele 2 bis 5 wurden auf dieselbe Art und Weise und in denselben Proportionen durchgeführt wie das Beispiel 1, wobei das Beispiel 5 mit Jod als Kontrastbeispiel gedacht wurde.

**Patentansprüche**

1. 2,2-Dichloracetoacetylchlorid der Formel

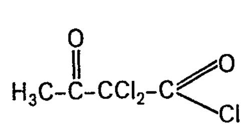

2. Verfahren zur Herstellung von 2,2-Dichloracetoacetylchlorid gemäss Patentanspruch 1, dadurch gekennzeichnet, dass Acetylchlorid mit

Hilfe einer Lewis-Säure als Katalysator mit Chlor bei einer Temperatur von 30 bis 60°C umgesetzt wird, wobei das Mol-Verhältnis von Acetylchlorid zum Katalysator und zum Chlor 1 zu 0,005 bis 0,5 zu 0,1 bis 1,5 beträgt.

3. Verfahren gemäss Patentanspruch 2, dadurch gekennzeichnet, dass als Katalysator Aluminiumhalogenid verwendet wird.

4. Verfahren gemäss Patentansprüchen 2 und 3, dadurch gekennzeichnet, dass als Katalysator Aluminiumchlorid verwendet wird.

5. Verfahren gemäss Patentansprüchen 2 und 3, dadurch gekennzeichnet, dass als Katalysator Aluminiumbromid verwendet wird.

6. Verfahren gemäss Patentansprüchen 2 bis 5, dadurch gekennzeichnet, dass die Chlorierungstemperatur 40 bis 50°C beträgt.

7. Verfahren gemäss Patentansprüchen 2 bis 6, dadurch gekennzeichnet, dass das Mol-Verhältnis von Acetylchlorid zum Katalysator und zum Chlor 1 zu 0,03 zu 1,02 beträgt.

**Revendications**

1. Chlorure de 2,2-dichloroacétoacétyle de formule

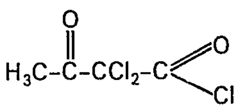

2. Procédé de préparation du chlorure de 2,2-dichloroacétoacétyle selon la revendication 1, caractérisé en ce que le chlorure d'acétyle est mis à réagir, à l'aide d'un acide de Lewis comme catalyseur, avec le chlore à une température de 30 à 60°C, le rapport molaire du chlorure d'acétyle au catalyseur et au chlore étant de 1 pour 0,005 à 0,5 pour 0,1 à 1,5.

3. Procédé suivant la revendication 2, caractérisé en ce qu'on utilise comme catalyseur un halogénure d'aluminium.

4. Procédé suivant les revendications 2 et 3, caractérisé en ce qu'on utilise comme catalyseur de chlorure d'aluminium.

5. Procédé suivant les revendications 2 et 3, caractérisé en ce qu'on utilise comme catalyseur du bromure d'aluminium.

6. Procédé suivant les revendications 2 à 5, caractérisé en ce que la température de chloration est de 40 à 50°C.

7. Procédé suivant les revendications 2 à 6, caractérisé en ce que le rapport molaire du chlorure d'acétyle au catalyseur et au chlore est de 1 pour 0,03 pour 1,02.

**Claims**

1. 2,2-dichloroaceto acetylchloride of the formula

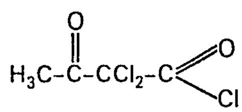

2. Process for preparing 2,2-dichloroaceto acetylchloride according to patent claim 1, characterized in that acetylchloride is reacted with chlorine at a temperature of 30 to 60°C with the aid of a Lewis-acid as catalyst, the molar ratio of acetylchloride to catalyst and to chlorine being 1:0.005 to 0.5:0.1 to 1.5.

3. Process according to patent claim 2, characterized in that aluminium halide is used as catalyst.

4. Process according to patent claims 2 and 3, characterized in that aluminium chloride is used as catalyst.

5. Process according to patent claims 2 and 3, characterized in that aluminium bromide is used as catalyst.

6. Process according to patent claims 2 to 5, characterized in that the temperature of chlorination is 40 to 50°C.

7. Process according to patent claims 2 to 6, characterized in that the molar ratio of acetylchloride to catalyst and to chlorine is 1:0.03:1.02.